# FASCICULE DE BREVET EUROPEEN

(11) **EP 2 590 632 B1**
(45) Date de publication et mention de la délivrance du brevet: **17.04.2019**
(21) Numéro de dépôt: 11743100.7
(22) Date de dépôt: 05.07.2011
(51) Int. Cl.: A61K 9/20, A61K 9/26, A61K 9/50, G01N 33/52

(54) **FORME PHARMACEUTIQUE POUR LUTTER CONTRE LA SOUMISSION CHIMIQUE D'UN MEDICAMENT**
PHARMAZEUTISCHE FORM ZUR BEKÄMPFUNG VON UNFREIWILLIGER INTOXIKATION MIT EINEM ARZNEIMITTEL
PHARMACEUTICAL FORM FOR COMBATING CHEMICAL SUBMISSION OF A MEDICAMENT

(30) Priorité: 06.07.2010 FR 1055491
(43) Date de publication de la demande: 15.05.2013
(73) Titulaire: ETHYPHARM, 92213 Saint-Cloud Cedex (FR)
(72) Inventeur: HERRY, Catherine, F-27670 Saint-Ouen du Tilleul (FR); CONTAMIN, Pauline, F-76220 La Feuillie (FR); DUPAU, Emmanuel, F-76100 Rouen (FR)
(74) Mandataire: Cabinet Plasseraud
(86) Numéro de dépôt international: PCT/FR2011/051601
(87) Numéro de publication internationale: WO 2012/007672

(56) Documents cités:
- EP-A1- 1 681 048
- EP-A2- 1 273 301
- WO-A1-00/38649
- WO-A2-03/026621
- WO-A2-2009/043844
- FR-A1- 2 829 932
- Anonymous: "Rohypnol", Connecticut Clearinghouse - A Program of Wheeler Clinic, 2003, pages 1-2, XP002628783, Extrait de l'Internet: URL:http://www.ctclearinghouse.org/topics/ customer-files/rohypnol.pdf [extrait le 2011-03-17]

## Description

L'invention a pour objet une forme pharmaceutique pour lutter contre la soumission chimique.

Depuis un certain nombre d'années les délinquants utilisent les propriétés hypnotiques de certaines molécules pour droguer quelqu'un à son insu. Les délinquants n'hésitent pas à introduire subrepticement dans la boisson de leur victime une forme pharmaceutique pour altérer le comportement de celle-ci, voire la rendre totalement incapable voire amnésique. Une fois la victime démunie de toute conscience, le délinquant peut profiter d'elle : vol, viol, extorsion de fonds. Par ailleurs, l'ingestion d'une telle forme pharmaceutique sans respect des doses prescrites peut engendrer de sérieuses conséquences, notamment si elle est absorbée avec une quantité d'alcool. La forme pharmaceutique peut également produire des interactions délétères avec d'autres médicaments que la victime aurait pris au préalable.

Il est connu de l'état de la technique un somnifère, le Rohypnol, largement utilisé à des fins illicites en raison de sa facilité de dissolution et de ses caractéristiques imperceptibles. La formulation de ce médicament a été révisée pour aboutir à un comprimé vert à l'extérieur et bleu à l'intérieur, pelliculé donc lent à fondre, dégageant une couleur bleu. Cependant, la coloration bleue n'est visible qu'après un quart d'heure d'immersion dans le liquide ; la victime n'est donc pas en mesure de détecter le somnifère introduit subrepticement si elle boit immédiatement sa boisson.

Il est également connu de l'état de la technique le document WO 2005/059541 qui porte sur un kit pour détecter les drogues introduites furtivement dans les boissons. Toutefois, ce système ne protège que les personnes munies de ce kit.

WO2009/043844 décrit des comprimés orodispersibles comprenant un opacifiant.

FR2829932 et WO2003/026621 décrivent un produit pharmaceutique administrable par voie orale, comprenant au moins un agent détrompeur de goût, de couleur et/ou d'aspect.

Le document WO2000/038649 décrit une composition pharmaceutique pour l'administration par voie orale destinée à éviter le détournement d'usage aux dépens d'un tiers, grâce à la flottaison du comprimé.

Il est donc impératif et urgent de trouver un système permettant de détecter immédiatement le détournement illicite de médicaments en cas de soumission chimique sans avoir recours à un dispositif ou à un kit de détection.

Un objectif essentiel de la présente invention est donc de proposer une forme pharmaceutique comprenant au moins un composé permettant la détection immédiate de ladite forme pharmaceutique introduite illicitement dans une boisson. Un autre objectif est de pouvoir détecter la forme pharmaceutique quelque soit la nature, la couleur de la boisson.

### BREVE DESCRIPTION DES FIGURES

La figure 1 est une photographie représentant un bécher contenant 250 mL d'eau (1) et un bécher dans lequel a été introduit un comprimé orodispersible de zolpidem comprenant 50 mg de silicate de calcium (agent opacifiant) (2) .

La figure 2 est une photographie représentant un verre de coca et un verre de coca dans lequel a été introduit un comprimé C1 orodispersible de zolpidem comprenant des particules flottantes.

La figure 3 est une photographie représentant un verre de coca dans lequel a été introduit un comprimé C2 orodispersible de zolpidem comprenant d'autres particules flottantes.

La présente invention décrit une nouvelle forme pharmaceutique pour lutter contre la soumission chimique. Ce but est atteint grâce à une formulation pharmaceutique comprenant un principe actif et au moins un composé permettant la modification immédiate des caractéristiques organoleptiques d'une boisson dans laquelle est introduite la forme pharmaceutique, ledit composé étant choisi dans le groupe comprenant : un agent opacifiant, un agent fluorescent, des particules flottantes, des particules perceptibles en bouche, des microgranules effervescents, et leurs mélanges.

L'invention décrit également une méthode pour lutter contre la soumission chimique comprenant :
- la mise en solution dans une boisson d'une forme pharmaceutique comportant un principe actif et au moins un composé permettant la modification immédiate des caractéristiques organoleptiques de ladite boisson choisi dans le groupe comprenant : un agent opacifiant, un agent fluorescent, des particules flottantes, des particules perceptibles en bouche, des microgranules effervescents, et leurs mélanges,
- la détection de la forme pharmaceutique par la modification immédiate des caractéristiques organoleptiques de la boisson.

L'invention décrit également l'utilisation dans une forme pharmaceutique, d'au moins un composé permettant la modification immédiate des caractéristiques organoleptiques d'une boisson choisi dans le groupe comprenant un agent opacifiant, un agent fluorescent, des particules flottantes, des particules perceptibles en bouche, des microgranules effervescents, et leurs mélanges, pour lutter contre la soumission chimique.

Selon la présente invention, par « soumission chimique » on entend l'administration à des fins criminelles ou délictuelles d'une substance psychoactive à l'insu de la victime.

L'invention a plus particulièrement pour objet une méthode pour la détection immédiate dans une boisson d'une forme pharmaceutique introduite illicitement dans ladite boisson, ladite méthode étant telle que définie à l'une quelconque des revendications 1 à 8.

### DESCRIPTION DETAILLEE DE L'INVENTION

La forme pharmaceutique décrite dans l'invention comprend un principe actif et au moins un composé permettant la détection immédiate de ladite forme pharmaceutique introduite illicitement dans une boisson. Selon l'invention, le composé est choisi parmi :
- les agents opacifiants, et/ou
- les agents fluorescents, et/ou
- les particules flottantes, et/ou
- les particules perceptibles en bouche, et/ou
- les microgranules effervescents.

Dans le cadre de l'invention, les composés peuvent être intégrés à la forme pharmaceutique individuellement ou en combinaison. On pourra par exemple réaliser une forme pharmaceutique contenant des particules flottantes, ou bien proposer une forme pharmaceutique comprenant un mélange des composés décrits ci-dessus.

La forme pharmaceutique est de préférence une forme pharmaceutique orale. Cependant il pourrait s'agir d'un autre type de forme pharmaceutique que le délinquant détournerait de sa destination première.

Dans la présente invention, on entend par "immédiate" la modification des caractéristiques organoleptiques de la boisson qui se produit en moins d'une minute, de préférence en moins de 30 secondes, plus préférentiellement encore en moins de 15 secondes à partir de l'introduction de la forme pharmaceutique dans la boisson.

Selon un autre aspect de l'invention, le terme "immédiate" peut également être défini comme la modification des caractéristiques organoleptiques de la boisson qui se produit en moins d'une minute, de préférence en moins de 30 secondes, plus préférentiellement encore en moins de 15 secondes à partir de l'introduction et l'agitation de la forme pharmaceutique dans la boisson. On entend par « agitation » une mise en mouvement du liquide, par exemple à l'aide d'une paille, cuillère, par mouvement du récipient.

### Les agents opacifiants

Les agents opacifiants sont des composés minéraux qui permettent de troubler les boissons. Il peut s'agir de silicates tels que le silicate de magnésium, le silicate d'aluminium (notamment le kaolin), le silicate de magnésium et d'aluminium, le silicate de calcium, le dioxyde de titane et leurs mélanges. Ces composés sont généralement présents au minimum en une quantité d'au moins 15 mg, de préférence de 15 à 100 mg, préférentiellement encore de 20 mg à 60 mg et plus préférentiellement encore de 25 à 40 mg. En dessous de 15 mg, l'opacité pourrait s'avérer plus difficilement détectable à l'oeil nu.

De façon avantageuse, les agents opacifiants intégrés dans une forme pharmaceutique orale permettent de troubler les boissons dans lesquelles ils sont introduits. Ces agents sont particulièrement intéressants pour troubler les boissons transparentes et claires comme l'eau, le vin blanc, le jus de pomme, les spiritueux tels que la vodka, le rhum blanc...

L'aspect opaque de la boisson apparait dès les premières secondes après introduction et l'agitation de la forme pharmaceutique dans ladite boisson.

### Les agents fluorescents

La forme pharmaceutique peut également comprendre un agent fluorescent en une quantité d'au moins 0,1 mg, de préférence en une quantité d'au moins 1 mg, préférentiellement encore entre 0,2 et 5 mg et plus préférentiellement encore entre 0,3 à 2 mg. Cet agent peut être la fluorescéine et ses dérivés, le vert d'indocyanine.

Cet agent est visible dans tous types de boissons en présence de rayonnements ultra-violets et dans l'obscurité. Il permet de révéler la forme pharmaceutique le contenant en émettant une lumière fluorescente qui se dégage de la boisson piégée. Cet agent est particulièrement utile pour avertir la victime lorsqu'elle se trouve dans un espace sombre où il est facile d'introduire furtivement un corps étranger dans une boisson.

### Les particules flottantes et les particules perceptibles en bouche

Selon un autre aspect de l'invention, la forme pharmaceutique peut comprendre des particules flottantes et/ou des particules perceptibles en bouche. Ces particules sont des microgranules comprenant un support neutre insoluble, ou rendu insoluble dans l'eau ou dans une solution alcoolique par enrobage avec un polymère insoluble ou par enrobage avec une matière lipidique.

### Microgranules

On entend par microgranules rendus insolubles dans l'eau ou dans une solution alcoolique, un support neutre constitué par des matériaux solubles dans l'eau ou dans une solution alcoolique recouverts d'au moins une couche de matériaux insolubles dans l'eau ou dans une solution alcoolique et dont la fonction est de limiter, voire d'empêcher la pénétration de ces dits milieux vers le coeur du support.

De façon avantageuse, le support neutre insoluble dans l'eau ou dans une solution alcoolique comprend au moins un excipient de nature hydrophobe choisi parmi : la cellulose, les dérivés de la cellulose (cellulose microcristalline), les dérivés des phosphates (phosphates de calcium), la silice et les dérivés des silicates (silicate de magnésium, silicate d'aluminium et leurs mélanges), la cire de carnauba.

Dans le cadre de la présente invention, un support neutre soluble dans l'eau ou dans une solution alcoolique peut également être utilisé. Le support neutre soluble peut comprendre au moins un excipient choisi parmi : l'amidon, le saccharose, les polyols tels que le mannitol ou le lactose et leurs mélanges.

Il est impératif que ce support neutre soluble soit rendu insoluble dans l'eau ou l'alcool en le recouvrant d'une couche d'enrobage de nature :
- soit polymérique comprenant au moins un polymère hydrophobe et éventuellement une charge inerte et/ou un agent plastifiant et/ou un agent tensio-actif,
- soit lipidique comprenant au moins une matière lipidique.

Dans le cadre de la présente invention, le support neutre insoluble peut également être recouvert par au moins une couche d'enrobage telle que décrite ci-dessus, dans la mesure où celle-ci n'augmente pas de façon rédhibitoire la densité des particules.

Le taux d'enrobage représente le rapport de la quantité de masse sèche constituant la couche d'enrobage sur la masse totale du microgranule avant enrobage (en masse sèche). Le taux d'enrobage est compris entre 0,1% à 50% m/m, de préférence, de 2% à 30% m/m, et, plus préférentiellement encore, de 5% à 40% m/m.

Le taux d'enrobage est tel que les particules obtenues ont une densité inférieure à celle de la boisson dans laquelle elles vont être introduites, de préférence une densité inférieure à 1, de telle sorte qu'elles restent à la surface de la boisson dans laquelle elles vont être introduites. De telles particules sont appelées particules flottantes.

### Couche d'enrobage polymérique :

Le polymère hydrophobe utilisé pour assurer le caractère insoluble des microgranules est sélectionné dans le groupe des produits suivants : les dérivés non hydrosolubles de la cellulose, les dérivés de (co)polymères (méth)acryliques, les dérivés des polyvinylacétates et leurs mélanges. Plus préférentiellement, le ou les polymère(s) hydrophobe(s) est (sont) choisi(s) dans le groupe de produits suivants : l'éthylcellulose, l'acétate butyrate de cellulose, l'acétate de cellulose, les copolymères ammonio-méthacrylates type A et type B vendus sous le nom commercial Eudragit®, notamment l'Eudragit® RS 30D, l'Eudragit NE 30D, l'Eudragit® RL 30D, l'Eudragit® RS PO et l'Eudragit® RL PO de la famille des poly(éthyl acrylate, méthyl méthacrylate, triméthylamonioéthyl méthacrylate), les polyvinylacétates et leurs mélanges.

La quantité de polymère hydrophobe est comprise entre 50% à 100%, de préférence de 70% à 100%, de la masse sèche de la couche d'enrobage.

Une charge inerte peut être présente dans la couche d'enrobage à raison de 0 à 50% m/m, de préférence de 0 à 20% m/m, et, plus préférentiellement encore, de 5 à 20% de la masse sèche du polymère hydrophobe d'enrobage.

La charge inerte uniformément répartie dans l'enrobage est choisie dans le groupe comprenant notamment le talc, la silice colloïdale anhydre, le stéarate de magnésium, le monostéarate de glycérol et leurs mélanges.

Lorsque l'enrobage est réalisé en voie aqueuse, un agent plastifiant peut être ajouté à la dispersion d'enrobage à raison de 0% à 50% m/m, de préférence, de 2% à 25% m/m, en masse sèche de polymère hydrophobe d'enrobage.

L'agent plastifiant est sélectionné notamment dans le groupe de produits suivants : le glycérol et ses esters, de préférence dans le sous-groupe suivant : les triglycérides à chaînes moyennes, les glycérides acétylés, glycéryl-monostéarate, glycéryl-triacétate, glycéryl-tributyrate, les phtalates, de préférence dans le sous-groupe suivant : dibutylphtalate, diéthylphtalate, diméthylphtalate, dioctylphtalate, les citrates, de préférence dans le sous-groupe suivant : acétyltributylcitrate, acétyltriéthylcitrate, tributylcitrate, triéthylcitrate, les sébaçates, de préférence dans le sous-groupe suivant : diéthylsébaçate, dibutylsébaçate, les adipates, les azélates, les benzoates, le chlorobutanol, les polyéthylène glycols, les huiles végétales, les fumarates, de préférence le diéthylfumarate, les malates, de préférence le diéthylmalate, les oxalates, de préférence le diéthyloxalate, les succinates ; de préférence le dibutylsuccinate, les butyrates, les esters de l'alcool cétylique, les malonates, de préférence le diéthylmalonate, l'huile de ricin (celle-ci étant particulièrement préférée), et leurs mélanges.

Plus préférentiellement, l'agent plastifiant est sélectionné dans le groupe de produits suivants : les monoglycérides acétylés notamment le Myvacet® 9-45, le triéthylcitrate (TEC), le dibutylsébaçate, la triacétine, et leurs mélanges.

L'agent tensioactif est optionnellement présent dans l'enrobage à raison de 0 à 30% m/m, de préférence de 0 à 20% m/m, et, plus préférentiellement encore, de 5 à 15% de la masse sèche de plastifiant. L'agent tensioactif est de préférence sélectionné dans le groupe de produits suivants : les sels alcalins ou alcalinoterreux des acides gras, le sodium dodécyl sulfate et le docusate de sodium étant préférés, les huiles polyoxyéthylénées, de préférence l'huile de ricin hydrogénée polyoxyéthylénée, les copolymères polyoxyéthylène-polyoxypropylène, les esters de sorbitan polyoxyéthylénés, les dérivés de l'huile de ricin polyoxyéthylénés, les stéarates, de préférence de calcium, de magnésium, d'aluminium ou de zinc, les polysorbates, les stéarylfumarates, de préférence de sodium, le béhénate de glycérol, le chlorure de benzalkonium, le bromure d'acétyltriméthyl ammonium, l'alcool cétylique et leurs mélanges.

### Couche d'enrobage lipidique :

Les microgranules peuvent également être enrobés par enrobage d'une matière lipidique.
La matière lipidique selon l'invention est sélectionnée notamment dans le groupe de produits suivants : glycéryl palmitostéarate, les cires, les polyoxylglycérides, le glycéryl béhénate.

La quantité de matière lipidique est comprise entre 50 et 100 %, de préférence entre 80 et 100% de la masse sèche de la couche d'enrobage.

La quantité de matière lipidique est choisie de telle sorte que la densité des particules résultantes soit inférieure à celle de la boisson dans laquelle elles vont être introduites, de préférence une densité inférieure à 1, de telle sorte qu'elles restent à la surface de la boisson dans laquelle elles vont être introduites.

Les particules flottantes présentent un diamètre total (support neutre optionnellement enrobé compris entre 50 et 500 µm, de préférence entre 200 et 500 µm afin de ne pas être perceptibles en bouche et assurer un certain confort pour le patient. En revanche, les particules perceptibles en bouche présentent quant à elles un diamètre total supérieur à 500.µm, de préférence supérieur à 1 mm de façon à être perçues par les lèvres et surtout par les papilles. La mesure du diamètre des particules flottantes et perceptibles en bouche est faite par granulométrie laser en voie sèche (granulomètre laser Malvern :mastersizer 2000).

De façon tout à fait avantageuse, lesdites particules perceptibles en bouche sont flottantes.

La quantité des particules flottantes et/ou perceptibles en bouche contenues dans la forme pharmaceutique est d'au moins 25 mg, de préférence 40 mg.

De façon préférée, les particules flottantes et/ou les particules perceptibles en bouche peuvent être colorées à l'aide d'au moins un agent colorant suivant : l'indigocarmine, l'érythrosine, le brillant bleu FCF, l'alphazurine FG, le fast green FCF, la quinzarine green SS, l'orange II, la tartrazine, Sunset yellow FCF et/ou peuvent être rendues fluorescentes à l'aide d'un agent fluorescent choisi dans le groupe comprenant la fluorescéine et ses dérivés et le vert d'indocyanine.

Avantageusement, le principe actif pourra également être coloré avec au moins un colorant tel que décrit ci-dessus afin d'empêcher le tri possible entre le principe actif et les particules flottantes et/ou les particules perceptibles en bouche.

Avantageusement encore, les particules flottantes et/ou perceptibles en bouche s'adaptent à tous types de boisson.

Dès l'introduction de la forme pharmaceutique dans la boisson, les particules flottantes remontent immédiatement à la surface de la boisson et sont visibles à l'oeil nu. Ces particules restent à la surface du liquide pendant au moins 5 minutes et de façon préférée pendant au moins 4 heures, plus préférentiellement pendant au moins 12 heures.

Les particules perceptibles en bouche, peuvent également être des particules flottantes. Elles sont quant à elles, détectées immédiatement lors de la prise de la première gorgée de la boisson piégée.

### Les microgranules effervescents

La forme pharmaceutique peut également comprendre des microgranules effervescents. Les microgranules effervescents comprennent un excipient basique qui créera une effervescence lorsqu'il sera en présence d'une boisson acide de type soda ou bière.

Selon un premier aspect, les microgranules comprennent un support neutre (soluble, insoluble ou rendu insoluble) enrobé par des particules d'un agent alcalin choisi dans le groupe comprenant le bicarbonate de sodium, le carbonate de calcium, et leurs mélanges.

La quantité d'agent alcalin est au moins supérieure à 5 mg, de préférence supérieure à 10mg et encore plus préférentiellement supérieure à 20mg.

Lorsque la forme pharmaceutique contenant les microgranules effervescents est introduite dans une boisson acide, les particules d'agent(s) alcalin(s) au contact de l'acide présent créent une effervescence visible à l'oeil nu.

Selon un mode de réalisation particulier de l'invention les microgranules effervescents peuvent être enrobés. L'enrobage est suffisamment perméable pour permettre la libération des particules d'agent effervescent sur une période d'au moins trente minutes à une heure. L'enrobage contient au moins un polymère insoluble de la famille des dérivés de la cellulose, des dérivés vinyliques ou des dérivés acryliques. Il peut comprendre un plastifiant et/ou un agent tensioactif. Il peut être perméabilisé par ajout d'un agent porogène soluble comme par exemple des dérivés solubles de la cellulose, la povidone, un agent désintégrant.

La quantité de microgranules effervescents contenus dans la forme pharmaceutique est d'au moins 25 mg, de préférence 40 mg.

De façon préférée, les microgranules effervescents peuvent être colorés à l'aide d'au moins un agent colorant choisi parmi : l'indigocarmine, l'érythrosine, le brillant bleu FCF, l'alphazurine FG, le fast green FCF, la quinzarine green SS, l'orange II, la tartrazine, Sunset yellow FCF et/ou peuvent être rendus fluorescents à l'aide d'un agent fluorescent choisi dans le groupe comprenant la fluorescéine et ses dérivés et le vert d'indocyanine.

Ainsi, une effervescence colorée et/ou effervescence apparaitra à la surface de la boisson après introduction de la forme pharmaceutique contenant les microgranules effervescents.

### Le principe actif

L'invention est adaptée pour tout principe actif qui modifie l'état de conscience du patient. Plus particulièrement, le principe actif est choisi dans le groupe comprenant : les anxyolytiques par exemple les benzodiazépines, les hypnotiques, les sédatifs, les analgésiques par exemple de type opioïde.

Les anxyolytiques sont une classe de médicaments psychotropes, de préférence choisi parmi l'Alprazolam, le Bromazépam, le Chlordiazépoxide, le Clobazam, le Clonazépam, le Clotiazépam, le Clorazépate, le Diazépam, l'Estazolam, le Flunitrazépam, le Lorazépam, le Lormétazépam, le Midazolam, le Nitrazépam, le Nordazépam, l'Oxazépam, le Prazépam, le Témazépam, le Tétrazépam, le Triazolam, la clozapine, l'olanzapine, la pirenzépine, le zolpidem, lezopiclone, le zaléplon, le méprobamate, l'étifoxine, et leurs mélanges.

Les opiodes sont de préférence choisi parmi l'Alfentanil, l'Aniléridine, le Butorphanol, le carfentanil, la Codéine, la Diamorphine (héroine), le Dextropropoxyphène, les Enképhalines, les Endorphines, le Fentanyl, l'Hydrocodone, l'Hydromorphone, la Méthadone, la Morphine, la Nalbuphine, l'Oxycodone,l'Oxymorphone, la Pentazocine, la Péthidine (mépéridine), le Propoxyphène, le Rémifentanil, la Sufentanil, le Tramadol et la Buprénorphine, et leurs mélanges.

Selon un aspect particulier de l'invention, le principe actif présent dans la forme pharmaceutique est sous forme solide.

Selon un mode de réalisation particulier, le principe actif pourra également être coloré à l'aide d'au moins un agent colorant. L'agent colorant peut être l'un de ceux décrits précédemment et/ou pourra être rendu fluorescent par ajout d'un agent fluorescent tel que décrit précédemment.

Selon un autre mode de réalisation, le principe actif pourra être monté sur les particules flottantes et/ou sur les particules perceptibles en bouche.

Selon un mode particulier de l'invention et outre les composés qui sont décrits plus haut et qui permettent de lutter contre la soumission chimique, la forme pharmaceutique peut comprendre également au sein de sa matrice au moins un agent colorant hydrosoluble choisi dans le groupe comprenant : l'indigocarmine ou E 132, l'érythrosine ou E 127, le brillant bleu FCF, l'alphazurine FG, le fast green FCF, la quinzarine green SS, l'orange II, la tartrazine, Sunset yellow FCF. Les agents colorants hydrosolubles pouvant être mis en oeuvre dans l'invention sont des colorants solubles dans tout liquide comprenant au moins en partie de l'eau et qui sont pharmaceutiquement acceptables.

L'agent colorant est présent en une quantité suffisante pour permettre une coloration assez intense pour être perçue à l'oeil nu et qui puisse apparaître dès les premières secondes après l'introduction et l'agitation de la forme pharmaceutique dans ladite boisson. Ainsi, l'agent colorant est présent à raison d'au moins 0,05 mg, de préférence de 0,2 à 5 mg, plus préférentiellement encore de 0,3 à 2 mg dans la forme pharmaceutique.

Lorsque l'agent colorant est de l'indigocarmine, une couleur bleu intense se dégage immédiatement de la forme pharmaceutique, colorant par exemple la boisson en bleu s'il s'agit d'une boisson incolore comme l'eau ou la limonade, ou en vert s'il s'agit d'une boisson jaune comme le jus d'orange.

L'érythrosine, autre agent colorant, colore les boissons en rouge.

### Boisson

Dans la présente demande, on utilisera le terme boisson pour désigner les boissons froides et les boissons chaudes, par exemple l'eau ; l'eau pétillante ; le vin (rouge, blanc, rosé) ; la bière (brune, blonde) ; les liqueurs ; les spiritueux tels que la vodka, le rhum, l'eau-de-vie, la téquila, le whisky ; les cocktails ; les jus de fruits tels que le jus d'orange, le jus de raisin ; les sodas tel que le coca, la limonade ; le café ; le thé. Ces boissons sont données à titre indicatif mais aucunement à titre limitatif.

Dans la présente invention, le récipient contenant une boisson dans laquelle la forme pharmaceutique peut être introduite a une contenance comprise entre 3 cl et 1 L.

### Le procédé de préparation

En fonction de la nature du principe actif, il peut être sous forme de microcristaux, de microgranules ou mis en suspension et monté sur un support neutre.

Lorsqu'il est monté sur un support neutre, le principe actif se trouve sous forme de solution ou suspension dans un solvant aqueux ou organique. Un agent liant, un diluant, un agent antistatique peuvent également être ajoutés.

Le support neutre peut être tout excipient inerte chimiquement et pharmaceutiquement, existant sous forme particulaire, cristalline ou amorphe. On cite à titre d'exemple des dérivés de sucres tels que le lactose, le saccharose, l'amidon hydrolysé (maltodextrines) ou encore des celluloses. Des mélanges tels que le saccharose et l'amidon ou à base de cellulose sont également utilisés pour la préparation de supports neutres sphériques.

Le principe actif peut également être mis sous forme de microgranules par un procédé en lui-même connu tel que, par exemple, l'extrusion-sphéronisation, le montage de principe actif en turbine perforée, en lit fluidisé et autres.

Une fois obtenus, ces microgranules sont éventuellement enrobés en turbine ou lit fluidisé.

Les différents procédés de fabrication des microgranules par granulation par voie sèche ou par voie humide, présentés dans « Remington's pharmaceutical Sciences, 16 ème Ed, 1980, Mack Publ. Co. of Easton, PA, USA,» peuvent être mis en oeuvre dans la présente invention.

Le principe actif peut être enrobé par un polymère choisi en fonction du type de libération souhaitée (immédiate, contrôlée, retardé) ou en fonction de ses propriétés de masquage de goût.

Le principe actif est ensuite combiné avec au moins un agent permettant de lutter contre la soumission chimique et avec au moins un excipient pharmaceutiquement acceptable.

Avantageusement, l'invention s'adapte à toute forme pharmaceutique, notamment à une forme orale, choisie parmi les comprimés non enrobés tels que les comprimés classiques, les comprimés à sucer, les comprimés sublinguaux, les comprimés à croquer, les comprimés effervescents, les comprimés dispersibles, les comprimés orodispersibles ; une poudre pour sachets ou gélules, les films fins.

L'invention est plus particulièrement utile pour des compositions pharmaceutiques à libération immédiate, car le malfaiteur souhaitera que l'effet de perte de vigilance soit le plus rapide possible. Elle pourrait cependant être adaptée à des formes à libération contrôlée.

L'homme du métier sait adapter la formulation en fonction de la forme pharmaceutique et de la libération souhaitées.

Les excipients pharmaceutiquement acceptables mis en oeuvre dans les compositions pharmaceutiques selon l'invention sont des excipients classiquement utilisés.

On peut citer à titre d'exemples:
- les liants : par exemple les dérivés de la cellulose tels que l'HPMC, en particulier les grades Pharmacoat® 603 et Pharmacoat® 606, ou l'hydroxypropylcellulose ou l'hydroxyéthylcellulose, la cellulose microcristalline, les dérivés de la polyvinylpyrrolidone, en particulier le grade PVP K 30, les dérivés du polyéthylène glycol, en particulier le polyéthylène glycol dont le poids moléculaire vaut entre 600 et 7000, tels que le PEG4000 et le PEG6000 notamment, et leurs mélanges, des dérivés vinyliques tels que le polyvinylalcool;
- les diluants : par exemple les diluants solubles tels que le lactose, le mannitol, les dérivés de la cellulose telle que la cellulose microcristalline;
- les conservateurs : par exemple les parabens, les anti-oxydants tel que l'acide ascorbique;
- les solubilisants : par exemple les poloxamers, les cyclodextrines;
- les délitants : par exemple la crospovidone, la croscarmellose sodique;
- les édulcorants : comme l'aspartam, l'acesulfam potassique;
- les lubrifiants : le stéarate de magnésium, le stéarylfumarate de sodium, l'huile de coton;
- les arômes : tels que l'arôme menthe, citron, cerise noire, etc;
- les tensioactifs : les sels alcalins ou alcalinoterreux des acides gras, le sodium dodécyl sulfate et le docusate de sodium, les huiles polyoxyéthylénées, de préférence l'huile de ricin hydrogénée polyoxyéthylénée, les copolymères polyoxyéthylène- polyoxypropylène, les esters de sorbitan polyoxyéthylénés, les dérivés de l'huile de ricin polyoxyéthylénés, les stéarates, de préférence de calcium, de magnésium, d'aluminium ou de zinc, les polysorbates, les stéarylfumarates, de préférence de sodium, le béhénate de glycérol, le chlorure de benzalkonium, le bromure d'acétyltriméthyl ammonium, l'alcool cétylique et leurs mélanges ;
- les agents d'écoulement (glidants) : par exemple la silice, le talc, leur mélange.

Dans le cadre de la présente invention, on entend par comprimé orodispersible un « comprimé multiparticulaire se désagrégeant dans la bouche au contact de la salive en moins de 40 secondes». Selon un mode de réalisation particulier, l'invention porte sur un tel comprimé qui est à base d'un mélange d'excipients et de particules de principe actif enrobé présentant des caractéristiques intrinsèques de compression. La proportion de mélange d'excipients par rapport aux particules de principe actif enrobé est de 0,4 à 6, de préférence de 1 à 4 parties en poids. Le mélange d'excipients comprend :
- un agent de désagrégation ou délitant;
- un agent soluble diluant à propriétés liantes;
- un lubrifiant;
- un composé permettant de lutter contre la soumission chimique choisi dans le groupe comprenant les agents opacifiants, les agents fluorescents, les particules flottantes, les particules perceptibles en bouche, les microgranules effervescents, et leurs mélanges;
- éventuellement un agent perméabilisant, des édulcorants, des arômes;
- et éventuellement un agent colorant permettant de lutter contre la soumission chimique,

La proportion d'agent de désagrégation et d'agent soluble par rapport à la masse du comprimé étant de 1 à 15%, de préférence de 2 à 7% en poids pour le premier et de 30 à 90%, de préférence de 40 à 70% en poids pour le second.

L'agent soluble diluant à propriétés liantes est constitué par un polyol de moins de 13 atomes de carbone se présentant soit sous la forme du produit directement compressible dont le diamètre moyen des particules est compris entre 100 et 500 micromètres, soit sous la forme d'une poudre dont le diamètre moyen des particules est inférieur à 100 micromètres, ce polyol étant de préférence choisi dans le groupe comprenant le mannitol, le xylitol, le sorbitol et le maltitol, le sorbitol ne pouvant être utilisé seul.

L'agent de désagrégation est choisi dans le groupe comprenant notamment la carboxyméthylcellulose sodique réticulée désignée dans le métier par le terme croscarmellose, la crospovidone et leur mélange. Grâce au choix et à la proportion de cet agent de désagrégation, le comprimé conserve une dureté acceptable pour des conditions de manipulation normales des comprimés lorsqu'ils sont conservés en conditionnement étanche jusqu'à des températures d'au moins 30° C.

Le lubrifiant préférentiellement utilisé dans ce mélange d'excipients est choisi dans le groupe comprenant le stéarate de magnésium, le stéarylfumarate de sodium, l'acide stéarique, le polyoxyéthylèneglycol micronisé (Macrogol 6000 micronisé), et leurs mélanges Il peut être utilisé dans une proportion de 0,05 à 2% par rapport à la masse totale du comprimé.

Comme agent perméabilisant on utilise un composé choisi dans le groupe comprenant notamment des silices ayant une grande affinité pour les solvants aqueux, telles que la silice précipitée plus connue sous le nom de marque Syloid, les maltodextrines, les 1-cyclodextrines et leurs mélanges.

L'agent perméabilisant permet la création d'un réseau hydrophile qui facilite la pénétration de la salive et contribue ainsi à une meilleure désagrégation du comprimé.

Les différents composés et procédés de fabrication des comprimés orodispersibles décrits dans FR2785538, WO0027357, FR2679451, WO 93/01805, FR2766089, WO 00/51568, FR2790387, WO 03/039520, FR2831820 peuvent être mis en oeuvre dans la présente invention.

L'invention va être décrite de façon plus détaillée ci-après, à l'aide des exemples qui sont donnés à titre d'illustration seulement.

### EXEMPLES

### Exemple 1

On prépare des comprimés orodispersibles comprenant 10 mg de zolpidem et un agent opacifiant présentant la composition suivante:

| constituants | % | mg/unité |
|---|---|---|
| Grains Zolpidem | 32,8 | 82,1 |
| Cellulose microcristalline | 9,6 | 23,90 |
| Mannitol | 30,0 | 75,00 |
| Crospovidone | 5,0 | 12,50 |
| Calcium silicate | 20,0 | 50,00 |
| Aspartam | 1,0 | 2,50 |
| Arôme | 0,1 | 0,25 |
| Silice | 1,0 | 2,50 |
| Mg stéarate | 0,5 | 1,25 |
| *Total* | *100,0* | *250,0* |

Les comprimés orodispersibles sont préparés de la façon suivante.

On prépare dans un premier temps les grains de zolpidem qui présentent la composition centésimale suivante :

| | |
|---|---|
| NPTAB 190 (180-220µm) | 56 |
| Zolpidem tartrate | 13 |
| Hypromellose 603 | 7 |
| HCl1N | 2 |
| Aquacoat ECD30 | 13 |
| Hypromellose 603 | 6 |
| Triéthyl citrate | 3 |

On dissout le zolpidem tartrate dans de l'eau à l'aide d'HCl, puis on prépare une dispersion par ajout d'hypromellose 603. Dans un lit fluidisé GPCG1 (Glatt), on introduit des sphères de sucre NPTAB 190 et la dispersion préparée ci-dessus. On introduit ensuite une dispersion aqueuse d'aquacoat ECD30, triéthyl citrate et hypromellose 603 pour obtenir un enrobage de masquage de goût.

Les grains de zolpidem sont ensuite mélangés aux excipients de compression. Le mélange pulvérulent est ensuite comprimé sur comprimeuse rotative (SVIAC PR12) dotée de poinçons ronds, convexes, à une force de compression de 5kN.

On obtient des comprimés de 250mg de diamètre 8,5mm qui présentent les caractéristiques suivantes:
Dureté: 38N
Désagrégation (mesurée selon monographie 2.9.1 de la pharmacopée européenne 6.1): 15 sec
Friabilité (mesurée selon monographie 2.9.7 de la pharmacopée européenne 6.1): 0,32%

Les comprimés sont agréables en bouche.

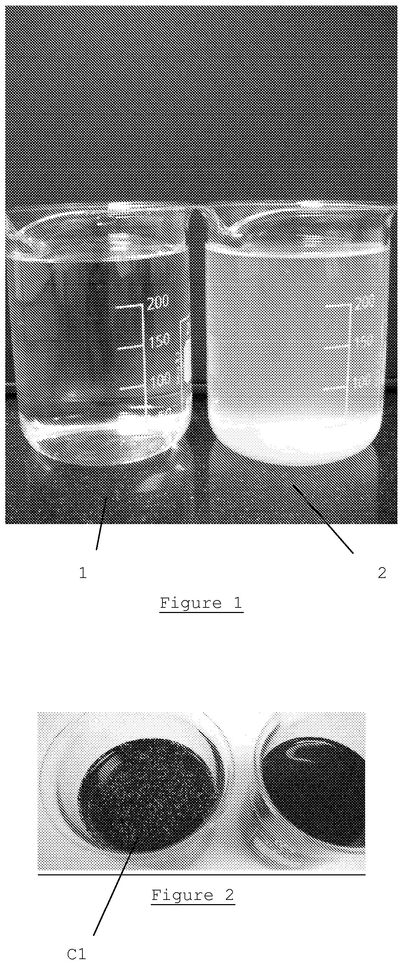

Un comprimé est introduit dans un récipient transparent contenant 250mL d'eau. Un trouble apparait dès que le comprimé est désagrégé, comme ceci est illustré sur la figure 1.

La figure 1 est une photographie représentant un bécher contenant 250mL d'eau (1) et un bécher dans lequel a été introduit le comprimé préparé dans cet exemple (2).

### Exemple 2

On prépare des comprimés classiques de zolpidem comprenant un agent opacifiant présentant la formule générale suivante :

| | % | Mg/unité |
|---|---|---|
| Grains Zolpidem | 32,8 | 82,0 |
| Cellulose microcristalline | 10,0 | 25,0 |
| Lactose | 32,7 | 81,75 |
| Calcium silicate | 20,0 | 50,0 |
| Povidone | 3,0 | 7,5 |
| Silice | 1,0 | 2,50 |
| Mg stéarate | 0,5 | 1,25 |
| *Total* | *100,0* | *250,0* |

Les grains de zolpidem sont préparés de la même façon que dans l'exemple 1 ci-dessus. Ils sont ensuite mélangés aux excipients et le mélange pulvérulent est comprimé.

Un comprimé ainsi préparé est introduit dans un verre d'eau dans lequel il se dissout en formant un trouble très visible à l'oeil nu.

### Exemple 3

On prépare deux types de comprimés orodispersibles comprenant des particules flottantes présentant la formule suivante:

| constituants | % | mg/unité |
|---|---|---|
| Grains Zolpidem | 32,8 | 82,1 |
| Cellulose microcristalline | 9,6 | 23,90 |
| Mannitol | 30,0 | 75,00 |
| Crospovidone | 5,0 | 12,50 |
| Particules flottantes | 20,0 | 50,00 |
| Aspartam | 1,0 | 2,50 |
| Arôme | 0,1 | 0,25 |
| Silice | 1,0 | 2,50 |
| Mg stéarate | 0,5 | 1,25 |
| *Total* | *100,0* | *250,0* |

Ces comprimés sont préparés comme dans l'exemple 1, à l'exception que l'on remplace le silicate de calcium par des particules flottantes.

Pour la première série de comprimés (C1) les particules flottantes sont préparées de la manière suivante:
Des neutres NPTAB 190 (180-220µm) sont enrobés avec une dispersion aqueuse d'éthylcellulose, triacétine et talc Le facteur d'enrobage est de 30% de masse sèche et le ratio talc/polymère est de 1 :2.

Pour la seconde série de comprimés (C2) les particules flottantes sont des particules de calcium phosphate dibasique dihydrate enrobées par glycéryl palmitostéarate Le rapport glycéryl palmitostéarate / phosphate de calcium dibasique dihydrate est 1 :4.

Les comprimés des deux séries se désintègrent en moins de 30s, et présentent une sensation agréable en bouche.

Un comprimé de chaque type est introduit dans un verre de coca cola. La désintégration se fait immédiatement et la présence de particules à la surface du coca cola est détectable à l'oeil nu. Ces particules flottantes sont visibles à la surface pendant plus de 3 heures.

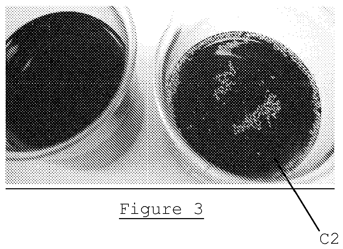

Les résultats sont présentés sur les figures 2 et 3 qui représentent respectivement une photographie d'un verre de coca et d'un verre de coca dans lequel a été introduit un comprimé C1 ou un comprimé C2. Les photographies ont été prises 5 minutes après l'introduction du comprimé. La présence des particules flottantes était néanmoins détectable beaucoup plus rapidement, environ 30 secondes après l'introduction des comprimés.

### Exemple 4

On prépare des gélules de morphine à libération immédiate comprenant des particules flottantes de la façon suivante:
Dans un lit fluidisé GPCG1 (Glatt), on introduit des neutres de sucre SP (400-600µm) et on pulvérise sur eux une dispersion aqueuse de sulfate de morphine et d'hypromellose 603, de façon à obtenir la composition centésimale suivante: 43% de neutre SP, 42% de sulfate de morphine et 15% d'hypromellose.

Séparément on prépare des particules flottantes de calcium phosphate dibasique dihydrate enrobées par glycéryl palmitostéarate comme mentionné dans l'exemple 3.

Dans des gélules on introduit 40mg de particules flottantes et la quantité nécessaire de granules de morphine pour obtenir le dosage souhaité 10mg, 20mg ou 30mg de morphine.

Les deux populations de particules présentes dans la gélule ne sont pas distinguables à l'oeil nu.

Lorsque le contenu d'une de ces gélules est introduit dans une boisson, les particules flottantes deviennent immédiatement apparentes à la surface de la boisson.

### Exemple 5

On prépare des gélules de sulfate de morphine comme dans l'exemple précédent mais en utilisant des neutres de diamètre 250-300 µm et en remplaçant les particules flottantes par des particules flottantes préparées de la façon suivante: Des neutres NPTAB (300-350µm) sont enrobés avec une dispersion aqueuse d'éthylcellulose, triacétine et talc. Le facteur d'enrobage est de 40% de masse sèche et le ratio talc/polymère est de 1 :2.

Les deux populations de particules sont introduites dans des gélules. Les deux populations de particules ne sont pas distinguables à l'oeil nu.

Le contenu d'une gélule est introduit dans une canette de coca-cola. Les particules flottantes remontent immédiatement à la surface et dès que la personne porte la boisson à la bouche, elle ressent la présence des microparticules, signe qu'une substance a été introduite dans la canette.

### Exemple 6

On prépare des comprimés orodispersibles comprenant 5 mg de zolpidem et un agent fluorescent présentant la composition suivante :

| | % | Mg/unité |
|---|---|---|
| Grains Zolpidem | 32,8 | 41,05 |
| Cellulose microcristalline | 10,0 | 12,50 |
| Mannitol | 43,7 | 54,57 |
| Crospovidone | 10,0 | 12,50 |
| Aspartam | 1,0 | 1,25 |
| Arôme | 0,1 | 0,13 |
| Fluorescéine | 0,4 | 0,50 |
| Silice | 1,0 | 1,25 |
| Mg stéarate | 1,0 | 1,25 |
| *Total* | *100,0* | *125,0* |

Les comprimés orodispersibles sont préparés de la façon suivante.

On prépare dans un premier temps les grains de zolpidem qui présentent la composition centésimale suivante :

| | |
|---|---|
| NPTAB 190 (180-220µm) | 56 |
| Zolpidem tartrate | 13 |
| Hypromellose 603 | 7 |
| HCl1N | 2 |
| Aquacoat ECD30 | 13 |
| Hypromellose 603 | 6 |
| Triéthyl citrate | 3 |

On dissout le zolpidem tartrate dans de l'eau à l'aide d'HCl, puis on prépare une dispersion par ajout d'hypromellose 603. Dans un lit fluidisé GPCG1 (Glatt), on introduit des sphères de sucre NPTAB 190 et la dispersion préparée ci-dessus. On introduit ensuite une dispersion aqueuse d'aquacoat ECD30, triéthyl citrate et hypromellose 603 pour obtenir un enrobage de masquage de goût.

Les grains de zolpidem sont ensuite mélangés aux excipients de compression. Le mélange pulvérulent est ensuite comprimé sur comprimeuse rotative (SVIAC PR12) dotée de poinçons ronds, convexes, à une force de compression de 5kN

On obtient des comprimés de 125mg de diamètre 7 mm qui présentent les caractéristiques suivantes:
Dureté: 24N
Désagrégation (mesurée selon monographie 2.9.1 de la pharmacopée européenne 6.1): 15 sec
Friabilité (mesurée selon monographie 2.9.7 de la pharmacopée européenne 6.1): 0,03%

Les comprimés sont agréables en bouche.

Un comprimé est introduit dans un récipient transparent contenant 250mL d'eau. La surface de l'eau devient fluorescente dès que le comprimé est désagrégé.

### Exemple 7

On prépare des comprimés orodispersibles comprenant 10 mg de zolpidem, des particules flottantes et un agent fluorescent présentant la composition suivante :

| | **mg/unité** | % |
|---|---|---|
| Gr. Zolpidem Tartrate* | 80.0 | 26.7 |
| Avicel PH 200 | 30.0 | 10.0 |
| Mannitol SD 200 | 99.7 | 33.2 |
| **Particules flottantes** | **50.0** | **16.7** |
| Kollidon CL | 30.0 | 10.0 |
| Aspartam | 3.0 | 1.0 |
| Arôme blackcherry | 0.3 | 0.1 |
| **Fluorescéine sodique** | **1.0** | **0.33** |
| Syloïd 244 FP | 3.0 | 1.0 |
| Stéarate Mg | 3.0 | 1.0 |
| Total | 300.0 | 100.0 |

Ces comprimés sont préparés comme dans l'exemple 1, à l'exception que l'on remplace le silicate de calcium par des particules flottantes et de la fluoréscéine sodique.

Les particules flottantes sont préparées de la manière suivante: des neutres NPTAB 190 (180-220µm) sont enrobés avec une dispersion aqueuse d'éthylcellulose et de Myvacet® (monoglycéride acétylé) 9-45. Le facteur d'enrobage est de 30% de masse sèche et le ratio plastifiant/polymère est de 24%.

Les comprimés se désintègrent en moins de 15s, et présentent une sensation agréable en bouche.

Un comprimé est introduit dans un verre de coca cola. La désintégration se fait immédiatement et la présence de particules à la surface du coca cola est détectable à l'oeil nu. Ces particules flottantes sont visibles à la surface pendant plus de 3 heures.

## Revendications

1. Méthode pour la détection immédiate dans une boisson d'une forme pharmaceutique introduite illicitement dans ladite boisson, ladite méthode comprenant :
* la mise en solution dans une boisson d'une forme pharmaceutique qui consiste en :
- un principe actif qui modifie l'état de conscience d'un patient,
- un agent opacifiant et des particules perceptibles en bouche et flottantes présentant une densité inférieure à 1 et un diamètre supérieur à 500µm, lesdites particules étant des microgranules comprenant un support neutre insoluble, ou rendus insolubles dans l'eau ou dans une solution alcoolique par enrobage avec un polymère insoluble ou par enrobage d'une matière lipidique,
et éventuellement avec un agent fluorescent,
- au moins un excipient pharmaceutiquement acceptable,
ladite forme pharmaceutique étant une forme pharmaceutique solide non enrobée se présentant sous forme de comprimé à croquer ou de comprimé orodispersible,
lesdits agent opacifiant, particules perceptibles en bouche et flottantes et éventuellement agent fluorescent permettant la modification immédiate des caractéristiques organoleptiques de la boisson dans laquelle est introduite ladite forme pharmaceutique,
* la détection de la forme pharmaceutique dans ladite boisson par la modification immédiate des caractéristiques organoleptiques de la boisson, ladite modification se produisant en moins d'une minute, de préférence en moins de 30 secondes, plus préférentiellement encore en moins de 15 secondes à partir de l'introduction de la forme pharmaceutique dans la boisson.

2. Méthode selon la revendication 1, **caractérisée en ce que** principe actif qui modifie l'état de conscience d'un patient est choisi dans le groupe comprenant : les anxyolytiques, les hypnotiques, les sédatifs, les analgésiques.

3. Méthode selon la revendication 1 ou 2, **caractérisée en ce que** l'agent opacifiant est un composé minéral choisi dans le groupe comprenant les silicates, tels que le silicate de magnésium, le silicate d'aluminium, le silicate de magnésium et d'aluminium, le silicate de calcium, le dioxyde de titane et leurs mélanges.

4. Méthode selon l'une quelconque des revendications 1 à 3, **caractérisée en ce que** l'agent opacifiant est présent en une quantité d'au moins 15 mg, de préférence de 15 à 100 mg, préférentiellement encore de 20 mg à 60 mg et plus préférentiellement encore de 25 à 40 mg.

5. Méthode selon l'une quelconque des revendications 1 à 4, **caractérisée en ce que** l'agent fluorescent est choisi dans le groupe comprenant la fluorescéine et ses dérivés et le vert d'indocyanine.

6. Méthode selon la revendication 5, **caractérisée en ce que** l'agent fluorescent est présent en une quantité d'au moins 0,1 mg, de préférence en une quantité d'au moins 1 mg, préférentiellement encore entre 0,2 et 5 mg et plus préférentiellement encore entre 0,3 à 2 mg.

7. Méthode selon l'une quelconque des revendications 1 à 6, **caractérisée en ce que** les particules flottantes et perceptibles en bouche sont présentes en une quantité d'au moins 25 mg, de préférence 40 mg.

8. Méthode selon l'une quelconque des revendications 1 à 7, **caractérisée en ce que** l'excipient pharmaceutiquement acceptable est choisi dans le groupe comprenant des liants, des diluants, des conservateurs, des solubilisants, des délitants, des édulcorants, des lubrifiants, des arômes, des tensioactifs, des agents d'écoulement et leurs mélanges.

## Patentansprüche

1. Verfahren zum sofortigen Nachweis in einem Getränk einer unerlaubt dem Getränk zugeführten Darreichungsform, wobei das Verfahren umfasst:
* Auflösen einer Darreichungsform in einem Getränk, bestehend aus:
- einem Wirkstoff, der den Bewusstseinszustand eines Patienten verändert,
- einem Trübungsmittel und im Mund wahrnehmbare und aufschwimmende Teilchen mit einer Dichte von weniger als 1 und einem Durchmesser von mehr als 500 µm, wobei die Teilchen Mikrogranulate sind, die einen unlöslichen neutralen Träger umfassen oder durch Beschichten mit einem unlöslichen Polymer oder durch Beschichten mit einem Lipidmaterial in Wasser oder in einer alkoholischen Lösung unlöslich gemacht wurden, und gegebenenfalls ein Fluoreszenzmittel,
- mindestens einen pharmazeutisch akzeptablen Hilfsstoff,
wobei die Darreichungsform eine unbeschichtete feste Darreichungsform ist, die in Form einer kaubaren Tablette oder einer orodispersiblen Tablette vorliegt,
wobei das Trübungsmittel, die im Mund wahrnehmbaren und aufschwimmenden Teilchen und gegebenenfalls das Fluoreszenzmittel die sofortige Veränderung der organoleptischen Eigenschaften des Getränks, dem die Darreichungsform zugeführt wurde, ermöglichen;
* Nachweisen der Darreichungsform im Getränk durch sofortige Veränderung der organoleptischen Eigenschaften des Getränks, wobei die Veränderung in weniger als einer Minute, vorzugsweise in weniger als 30 Sekunden, stärker bevorzugt in weniger als 15 Sekunden, ab dem Zeitpunkt der Zuführung der Darreichungsform zu dem Getränk erfolgt.

2. Verfahren gemäß Anspruch 1, **dadurch gekennzeichnet, dass** der Wirkstoff, der den Bewusstseinszustand eines Patienten verändert ausgewählt ist aus der Gruppe umfassend: Anxiolytika, Hypnotika, Sedativa, Analgetika.

3. Verfahren gemäß Anspruch 1 oder 2, **dadurch gekennzeichnet, dass** das Trübungsmittel eine anorganische Verbindung ist, ausgewählt aus der Gruppe umfassend Silikate, wie Magnesiumsilicat, Aluminiumsilicat, Magnesium- und Aluminiumsilicat, Calciumsilicat, Titandioxid und Mischungen davon.

4. Verfahren gemäß einem der Ansprüche 1 bis 3, **dadurch gekennzeichnet, dass** das Trübungsmittel in einer Menge von mindestens 15 mg, vorzugsweise 15 bis 100 mg, stärker bevorzugt 20 mg bis 60 mg und besonders bevorzugt 25 bis 40 mg vorliegt.

5. Verfahren gemäß einem der Ansprüche 1 bis 4, **dadurch gekennzeichnet, dass** das Fluoreszenzmittel ausgewählt wird aus der Gruppe umfassend Fluorescein und Derivate davon und Indocyaningrün.

6. Verfahren gemäß Anspruch 5, **dadurch gekennzeichnet, dass** das Fluoreszenzmittel in einer Menge von mindestens 0,1 mg, vorzugsweise in einer Menge von mindestens 1 mg, stärker bevorzugt zwischen 0,2 und 5 mg und besonders bevorzugt zwischen 0,3 und 2 mg vorliegt.

7. Verfahren gemäß einem der Ansprüche 1 bis 6, **dadurch gekennzeichnet, dass** die im Mund wahrnehmbaren und aufschwimmenden Teilchen in einer Menge von mindestens 25 mg, vorzugsweise 40 mg, vorliegen.

8. Verfahren gemäß einem der Ansprüche 1 bis 7, **dadurch gekennzeichnet, dass** der pharmazeutisch akzeptable Hilfsstoff ausgewählt ist aus der Gruppe umfassend Bindemittel, Verdünnungsmittel, Konservierungsmittel, Lösungsvermittler, Sprengmittel, Süßungsmittel, Gleitmittel, Geschmacksstoffe, Tenside, Fließmittel und Mischungen davon.

## Claims

1. A method for the immediate detection into a drink of a pharmaceutically form illicitly introduced into said drink, said method comprising:
* dissolution in a drink of a pharmaceutical form consisting of:
- an active principle which modifies the state of consciousness of a patient,
- an opacifying agent and particles perceptible in the mouth and floating having a density less than one and a diameter greater than 500 µm, said particles being microgranules comprising a blank support which is insoluble or rendered insoluble in water or in an alcoholic solution by coating with an insoluble polymer or by coating with a lipid material,
and possibly a fluorescent agent,
- at least one pharmaceutically acceptable excipient,
said pharmaceutical form being a solid non-coated pharmaceutical form presented in the form of chewable tablet or orodispersible tablet,
said opacifying agent, particles perceptible in the mouth and floating and possibly fluorescent agent enabling the immediate modification of the organoleptic properties of the drink into which said pharmaceutical form is introduced,
* detection of the pharmaceutical form in said drink through the immediate modification of the organoleptic properties of the drink, said modification taking place in less than one minute, preferably in less than 30 seconds, still more preferably in less than 15 seconds, from the introduction of the pharmaceutical form in the drink.

2. The method according to claim 1, **characterized in that** the active principle which modifies the state of consciousness of a patient is selected from the group comprising: anxiolytics, hypnotics, sedatives and analgesics.

3. The method according to claim 1 or claim 2, **characterized in that** the opacifying agent is an inorganic compound selected from the group comprising silicates, such as magnesium silicate, aluminum silicate, magnesium aluminum silicate, calcium silicate, titanium dioxide and mixtures thereof.

4. The method according to any one of claims 1 to 3, **characterized in that** the opacifying agent is present in a quantity of at least 15 mg, preferably from 15 to 100 mg, more preferably from 20 mg to 60 mg and still more preferably from 25 to 40 mg.

5. The method according to any one of claims 1 to 4, **characterized in that** the fluorescent agent is selected from the group comprising fluorescein or derivatives thereof and indocyanine green.

6. The method according to claim 5, **characterized in that** the fluorescent agent is present in a quantity of at least 0.1 mg, preferably at least 1 mg, more preferably between 0.2 and 5 mg and more preferably between 0.3 and 2 mg.

7. The method according to any one of claims 1 to 6, **characterized in that** the floating particles and perceptible in the mouth are present in a quantity of at least 25 mg, preferably 40 mg.

8. The method according to any one of claims 1 to 7, **characterized in** the pharmaceutically acceptable excipient is selected from the group comprising binders, diluents, preservatives, solubilizers, disintegrants, sweeteners, lubricants, flavors, surfactants, glidants and mixtures thereof.
